# EUROPEAN PATENT APPLICATION

(11) **EP 1 415 657 A1**
(43) Date of publication of application: **06.05.2004**
(21) Application number: 02746002.1
(22) Date of filing: 12.07.2002
(51) Int. Cl.: A61K 35/78, A61K 31/12, A61P 25/28, A61P 43/00, A23L 1/30, A23L 2/38, A23K 1/18

(54) **REMEDIES**

(30) Priority: 13.07.2001 JP 2001213255
(71) Applicant: TAKARA BIO INC., Otsu-shi, Shiga 520-2193 (JP)
(72) Inventor: NISHIYAMA, Eiji, Moriyama-shi, Shiga 524-0102 (JP); DEGUCHI, Suzu, Otsu-shi, Shiga 520-2134 (JP); OHNOGI, Hiromu, Kusatsu-shi, Shiga 525-0025 (JP); SUGIYAMA, Katsumi, Otsu-shi, Shiga 520-2134 (JP); SAGAWA, Hiroaki, Kusatsu-shi, Shiga 525-0025 (JP); KATO, Ikunoshin, Shiga 529-1851 (JP)
(74) Representative: VOSSIUS & PARTNER
(86) International application number: PCT/JP2002/007095
(87) International publication number: WO 2003/006037

(57) **Abstract**

According to the present invention, there is provided a medicament for oral administration or intravenous administration that is effective for a disease requiring enhancement of nerve growth factor production, comprising as an effective ingredient a pulverized product and/or extract obtained from *Humulus lupulus* and/or *Angelica keiskei* koidz., or xanthohumol and/or a pharmacologically acceptable salt thereof. 'The medicament is useful as a therapeutic agent or prophylactic agent for a disease requiring enhancement of nerve growth factor production, such as dementia or a nerve disorder.

Also, according to the present invention, there are provided an enhancing agent for nerve growth factor production, and a food, beverage or feed for enhancing nerve growth factor production, each comprising the above effective ingredient. By taking the food or beverage as foodstuff on a daily basis, the symptoms of a disease requiring enhancement of nerve growth factor production can be ameliorated or the like. In addition, the feed is useful for maintaining homeostasis of a living body by its action of enhancing nerve growth factor production.

Furthermore, these medicament and the like of the present invention are effective for improving or ameliorating learning and memorizing ability of an individual organism.

## Description

### TECHNICAL FIELD

The present invention relates to a medicament for enhancing nerve growth factor production used for oral administration or intravenous administration, comprising as an effective ingredient a pulverized product and/or extract of a plant, or a food, beverage or feed for enhancing nerve growth factor production, comprising the pulverized product and/or extract.

### BACKGROUND ART

*Angelica keiskei* koidz. is a large-scaled perennial plant belonging to Umbelliferae which has been used as a food or medicinal herb from the old days. As pharmacological actions owned by *Angelica keiskei* koidz., prophylactic effect for hypertension, antibacterial action, anti-ulcerative action, suppressive action for gastric acid secretion, anti-cancerous effect, prophylactic effect for cancer and the like have been known. On the other hand, *Humulus lupulus* is a lianoid perennial plant used for improving flavor and bitterness during the beer brewery. As pharmacological actions owned by *Humulus lupulus,* antibacterial action, anti-tumor action, sedative action and the like have been known. In addition, xanthohumol is a kind of chalcone contained in *Humulus lupulus,* and various physiological activities have been recently studied. However, so far, the enhancing actions for nerve growth factor production of these plants and xanthohumol have not been known at all.

Nerve cells play a principal role for sustaining psychoactivities of human being such as intellectual functions, memory, emotions and behaviors. It has been thought that the differentiation, survival and exhibition of functions of the nerve cells which are the foundations of these psychoactivities need a neurotrophic factor specific for each nerve cell. Among the neurotrophic factors, one of which existence and function have been firstly elucidated is a nerve growth factor (hereinafter simply referred to as "NGF") in some cases, and currently, there have been found a brain-derived-neurotrophic factor, neurotrophin-3, neurotrophin-4/5, and the like.

NGF is a neurotrophic factor of a large cellular cholinergic nerve cell of basal portion of the forebrain, so that its association with Alzheimer's dementia has been remarked [*Pharmacia*, Vol.22, No.2, 147-151 (1986), *Ronen Seishin Igaku (Senile Psychiatry),* Vol.3, No.6, 751-758 (1986)].

Alzheimer's dementia refers to a disease that gives a pathological finding such as senile plaque or Alzheimer's fibrillar changes, which are accompanied by a clinical picture such as developmental disability, manic state, tonic seizures of lower limbs, or epileptic seizure, and is one disease of senile dementia. The Alzheimer's dementia tends to be increasing in recent aging society, so that a larger societal interest has been drawn thereto. However, there has not yet been found a satisfactory method for ameliorating or treating such symptoms. There has not yet been also found such a method for juvenile Alzheimer's diseases.

In the brain of a patient with Alzheimer's dementia, there has been found a dramatic denaturation, a drastic lowering of the activity of choline acetyl transferase (CAT), in the basal portion of the forebrain centering about Meynert's basal nuclei [*Annu. Rev. Neurosci*., Vol.3, 77 (1980)]. In the studies of a rat brain in 1985, there has been elucidated that NGF is a neurotrophic factor at this site of the brain [*EMBO J.,* Vol.4, 1389 (1985)], so that the association of NGF with this disease has been remarked. In addition, there have been elucidated that in the striate body of the brain of a patient with Huntington's chorea, there are remarkable detachment of GABAergic nerve cell as well as' detachment of cholinergic nerve cell, so that NGF also acts on the endogenous cholinergic nerve cell of the striate body [*Science,* Vol.234, 1341 (1986)], addressing a possibility that this disease is associated with NGF. The effects of NGF have been studied with an animal such as a rat which can serve as a model for various nerve diseases. There has been reported that the degeneration of the nerve cell can be stopped in a rat if NGF is intracerebrally administered before the degeneration becomes remarkable, and that the lowering of CAT activity is also prevented [*J. Neurosci*., Vol.6, 2155 (1986), *Brain Res*., Vol.293, 305 (1985), *Science,* Vol.235, 214 (1986), *Proc. Natl. Acad. Sci. USA*, Vol.83, 9231 (1986)]. Also, it has been proven that NGF is biosynthesized in the peripheral sympathetic nerve-dominant tissues and in the brain, and that each of fibroblasts or astroglia which are interstitial cells for peripheral tissues or brain tissues plays an important role for the NGF biosynthesis [*J. Biol. Chem*., Vol.259, 1259 (1984), *Biochem. Biophys. Res. Commun*., Vol.136, 57 (1986)]. In addition, it has been elucidated that antigenicity, molecular weight, isoelectric point and biological activity of the fibroblast-produced or astroglia-produced NGF are the same as NGF of conventionally well studied submandibular gland. At the same time, it has been found that a catecholamine such as norepinephrine, epinephrine or dopamine shows enhancing effect for NGF production by a test of adding various neurotransmitters to a culture medium of fibroblasts (L-M cells) and astroglia [*J. Biol. Chem*., Vol.201, 6039 (1986)].

As described above, there has been expected that NGF can be used as a therapeutic agent for stopping degeneration in a nerve disease in which a site at which NGF acts as a neurotrophic factor is degenerated. In addition, once the cranial nerve cells are degenerated by cebrovascular disorders, cerebral tumor, cerebral apicitus, head injury, nerve degenerative disease, intoxication with an anesthetic, or the like, the degenerated cranial nerve cells would never recover during the life time, whereby various disorders such as emotional disorders and behavioral abnormality are consequently caused in addition to lowering in the intellectual functions and memory disabilities. On the other hand, nerve fiber shows plasticity, that is, when the nerve fiber is damaged, budding takes place from its surrounding healthy fibers, so that a new synapsis is formed in place of the damaged synapsis. Therefore, it has been expected that NGF can be used as a therapeutic agent for promoting restoration and regeneration of nerve functions at this stage.

However, when NGF is applied to a treatment of various nerve diseases, NGF must reach in very close vicinity of nerve cell that requires NGF, and NGF must be also transmitted to lesion site of the brain cell in a case of a disease in the central nervous system. However, NGF cannot be transmitted into the brain through the blood system. This is because the vascular endothelial cells in the brain are bound to each other by adhesion bonding (referred to as brain blood barrier), so that there is a limitation in the transport of a substance other than water, gas or a fat-soluble substance from blood to a brain tissue, whereby a protein (including NGF), which is polymeric substance, cannot pass through the brain blood barrier. There is a too large risk involved in the introduction of NGF directly into the brain by a surgical means, even if the introduction is conducted by the current techniques.

On the other hand, there has been developed a substance for enhancing NGF production, not a direct administration of NGF. Most of the compounds, however, have various problems such that the compounds have strong toxicity, or the compounds have effective concentration very closely approximating concentration at which toxicity is shown, or the compounds have severe adverse actions against nervous system such as nerve excitation action. Therefore, these compounds have not yet been actually used.

### DISCLOSURE OF INVENTION

An object of the present invention is to provide a medicament, food, beverage or feed, comprising as an effective ingredient a pulverized product and/or extract obtained from *Humulus lupulus* and/or *Angelica keiskei* koidz. having enhancing action for nerve growth factor production.

Summarizing the present invention, a first invention of the present invention relates to a therapeutic agent or prophylactic agent for oral administration or intravenous administration for a disease requiring enhancement of nerve growth factor production for treatment or prevention, characterized in that the therapeutic agent or prophylactic agent comprises as an effective ingredient a pulverized product and/or extract obtained from *Humulus lupulus* and/or *Angelica keiskei* koidz.

A second invention of the present invention relates to a therapeutic agent or prophylactic agent for oral administration or intravenous administration for a disease requiring enhancement of nerve growth factor production for treatment or prevention, characterized in that the therapeutic agent or prophylactic agent comprises as an effective ingredient xanthohumol and/or a pharmacologically acceptable salt thereof.

Third and fourth inventions of the present invention relate to an enhancing agent for nerve growth factor production for oral administration or intravenous administration, characterized in that the enhancing agent comprises the effective ingredient of the first or second invention of the present invention.

Fifth and sixth inventions of the present invention relate to a food, beverage or feed for enhancing nerve growth factor production, characterized in that the food, beverage or feed comprises the effective ingredient of the first or second invention of the present invention.

Seventh and eighth inventions of the present invention relate to an agent for improving or ameliorating learning and memorizing ability, characterized in that the agent comprises the effective ingredient of the first or second invention of the present invention.

Ninth and tenth inventions of the present invention relate to a food, beverage or feed for improving or ameliorating learning and memorizing ability, characterized in that the food, beverage or feed comprises the effective ingredient of the first or second invention of the present invention.

In the first, the third, the fifth, the seventh and the ninth inventions of the present invention, the pulverized product and/or extract obtained from *Humulus lupulus* includes the pulverized product and/or extract characterized in that the pulverized product and/or extract containing xanthohumol and/or a pharmacologically acceptable salt thereof in a high content.

### BEST MODE FOR CARRYING OUT THE INVENTION

In the present specification, the term "enhancing activity for NGF production" and "enhancing action for NGF production" each refers to a function for enhancing NGF production and enhancement of NGF production, and is not intended to particularly strictly distinguish in its meaning. In addition, the term "enhance" encompasses an embodiment in which the amount of the desired substance is increased after the action as compared to that before the action of the effective ingredient according to the present invention, as well as an embodiment in which the desired substance is produced by the action of the effective ingredient according to the present invention (induce). Also, in the present specification, any of the substances listed as effective ingredient can be used alone or in admixture of two or more kinds in the present invention.

The pulverized product and/or extract obtained from *Humulus lupulus* and/or *Angelica keiskei* koidz. usable as an effective ingredient in the present invention is not particularly limited as long as the pulverized product and/or extract is those which can exhibit an enhancing activity for nerve growth production by oral administration or intravenous administration, for instance, those which can be confirmed for the exhibition of the enhancing action of nerve growth factor production in accordance with the process as described in item (2) of Example 3 set forth below. In other words, when the pulverized product and/or extract is orally administered or intravenously administered to an organism individual, the pulverized product and/or extract may be those which can be confirmed for the increase in the amount of nerve growth factor production in a given tissue of an administered individual, as compared to those without administration. Also, the pulverized product and/or extract according to the present invention is not particularly limited as long as the pulverized product and/or extract can be obtained by the process for preparing the pulverized product and/or extract described below.

The term "effective ingredient" as used herein means the effective ingredient itself. Concretely explaining the term, although the extract used as an effective ingredient may be, for instance, used as a mixture with an extraction solvent in actual use, the "extract" does not mean the mixture, but the extract itself. Therefore, the extract used as an effective ingredient means the extract itself (i.e. dried product of the extract itself). In addition, an organism individual or individual refers to an organism such as human, or culturing or breeding animals given below.

In the present invention, pulverization refers to disintegration of solid or powder by dynamic forces such as impact, compression, shearing or friction. In other words, the pulverized product in the present invention refers to a product obtained by pulverizing a plant of *Humulus lupulus* and/or *Angelica keiskei* koidz. or a processed product thereof (for instance, dried product, frozen product or the like). The portion of the plant which can be used in the pulverization is not particularly limited. It is preferable to use root, stem, leaf or a mixture thereof, from the viewpoint of obtaining an excellent exhibition of the desired effects of the present invention. As the pulverization process, the pulverization can be carried out by using, for instance, a pulverizer. For instance, rough pulverizer, intermediate pulverizing apparatus, fine pulverizing apparatus, or ultrafine pulverizing apparatus, freezing-pulverizing apparatus or the like can be used. In addition, the pulverization process may be carried out by hand operation without using a pulverizer.

The form of the pulverized product is not particularly limited, and the pulverized product may be any of the forms of powder, sol or solid. The resulting pulverized product can be used in the form of a granular solid prepared by, for instance, granulating the pulverized product by a known process. The granulation process is not particularly limited, and is exemplified by tumbling granulation, agitation granulation, fluidizing bed granulation, airflow granulation, extruding granulation, compression molding granulation, disintegration granulation, spray granulation, spray-drying granulation or the like. In addition, the powdery pulverized product can be used in the present invention in the form of a liquid prepared by, for instance, dissolving or suspending a powdery pulverized product in a liquid, for instance, water, an alcohol or the like. The enhancing activity for NGF production of the pulverized product can be conveniently evaluated in accordance with the process described in item (2) of Example 3 set forth below.

A process for preparing an extract of *Humulus lupulus* and/or *Angelica keiskei* koidz. usable in the present invention includes the following known method. For instance, fruit, seed, seed coat, flower, leaf, stem, root and/or rhizome or the like, of the raw material *Humulus lupulus* and/or *Angelica keiskei* koidz. is collected in an appropriate timing, and thereafter used directly or usually subjected to a drying process such as an air-drying and then pulverized as desired, to give a raw material for extraction. Also, the raw material may be aged under various conditions, and the aged raw material may be used. When the raw material is a squeezed juice of a plant, the raw material can be directly used as a raw material for extraction. The extraction can be carried out with a solvent in a batch process or a continuous process. Furthermore, the squeezed juice of *Humulus lupulus* and/or *Angelica keiskei* koidz. can be directly used as an extract.

In the present invention, the extract refers to a substance extracted from *Humulus lupulus* and/or *Angelica keiskei* koidz. through the step of an extraction procedure with an extraction solvent, or a squeezed juice of *Humulus lupulus* and/or *Angelica keiskei* koidz.

The extraction solvent includes hydrophilic or lipophilic solvents such as water, chloroform, alcohols such as ethanol, methanol and isopropyl alcohol, ketones such as acetone and methyl ethyl ketone, methyl acetate, and ethyl acetate. These solvents can be used alone or appropriately as a mixed solution as desired. Usually, the extraction method is preferably carried out with water, an ethanol-containing water, or ethanol. The amount of the extraction solvent may be appropriately determined, and the extraction solvent may be used in an amount of preferably from 1 to 100 times the weight of the raw material as calculated by the dried product. The extraction temperature may be also appropriately determined according to its purpose. In the case of a water extraction, usually the extraction temperature is preferably from 4° to 130°C, more preferably from 25° to 100°C. In addition, when ethanol is contained in the solvent, the extraction temperature is preferably within the range of from 4° to 60°C. The extraction time may be also determined in consideration of the extraction efficiency. Usually, the raw material, the extraction solvent, and the extraction temperature are preferably set in consideration of the extraction efficiency so that the extraction time is preferably from several minutes to several days, more preferably from 5 minutes to 3 hours. The extraction procedure may be carried out with stirring or allowing the mixture to stand still, and the extraction procedure may be repeated several times as desired. The extract may be subjected to such a treatment as filtration, centrifugation, concentration, ultrafiltration, or molecular sieving as desired. The enhancing activity for NGF production of the extract can be conveniently evaluated by the method described in item (2) of Example 3 set forth below.

The extract can take any of the forms of powders, solids and liquids by carrying out a known processing treatment after the extraction. In addition, the extract can be made into a powdery form, and granulated in the same manner as the pulverized product described above.

In addition, the fraction obtained by fractionating the extract by a known method can be used as the extract of the present invention, as long as the fraction has an enhancing action for NGF production of the present invention. The fractionation means include extraction, separation by precipitation, column chromatography, thin-layer chromatography, and the like. The enhancing substance for NGF production can also be isolated by further proceeding the purification of the resulting fraction using the enhancing activity for NGF production as an index. In addition, an extract obtained by processing *Humulus lupulus* and/or *Angelica keiskei* koidz. into a tealeaf form by a known method, and extracting with the tealeaves can be used as the extract of the present invention, as long as the extract has an enhancing activity for NGF production. These extracts can be each used alone or in admixture of two or more kinds. Incidentally, in the present invention, extracts obtained by different extraction methods from the same plant can be each used alone or in an admixture of two or more kinds.

In the present invention, as the pulverized product and/or extract of *Humulus lupulus,* a pulverized product and/or extract of *Humulus lupulus* containing xanthohumol and/or a pharmacologically acceptable salt thereof in a high content can be used. Xanthohumol is a kind of chalcone uniquely contained in *Humulus lupulus*.

The term "high content" as referred to herein means that the content of xanthohumol (weight) per unit weight of the pulverized product and/or extract of *Humulus lupulus* [content ratio (% by weight) of xanthohumol in pulverized product and/or extract of *Humulus lupulus*] is higher than the content of xanthohumol (weight) per unit weight of the raw material *Humulus lupulus* [content ratio (% by weight) of xanthohumol in *Humulus lupulus*], i.e. xanthohumol is more condensed in its pulverized product and/or extract than that of the raw material *Humulus lupulus.* Concretely, the high content refers to a content ratio of xanthohumol in the pulverized product and/or extract of *Humulus lupulus* of preferably 1.5-folds or more, more preferably 2-folds or more, even more preferably 3-folds or more that of a content ratio of xanthohumol in *Humulus lupulus.* In addition, the term "high content" means that, as concretely expressed by a content ratio of xanthohumol in the pulverized product and/or extract of *Humulus lupulus,* xanthohumol is contained in the pulverized product and/or extract in an amount of preferably 0.05% by weight or more, more preferably 0.1% by weight or more. The same can be said for the pharmacologically acceptable salt of xanthohumol.

The pulverized product of *Humulus lupulus* containing xanthohumol in a high content can be easily obtained by, for instance, pulverizing *Humulus lupulus* by the above-mentioned pulverization process, and drying the pulverized product, to give a dry powder. Preferably, a dry powder may be prepared using as a raw material a tissue containing xanthohumol in a relatively high content, for instance, using one mainly comprising flower portions. In addition, the extract of *Humulus lupulus* containing xanthohumol in a high content can be obtained by, for instance, appropriately concentrating an extract obtained from *Humulus lupulus,* or fractionating the extract, thereby giving a fraction having a high enhancing activity for NGF production. The preparation of the pulverized product and extract containing the pharmacologically acceptable salt of xanthohumol in a high content can be carried out by preparing each of the desired pulverized product and extract in the coexistence of an alkali metal salt or the like mentioned below.

In addition, in the present invention, xanthohumol and/or a pharmacologically acceptable salt thereof can be directly used as an effective ingredient. Xanthohumol can be purified from *Humulus lupulus* by a known purification process. Also, a commercially available purified product can be used.

On the other hand, in the present invention, as the pulverized product and/or extract *of Angelica keiskei* koidz., there can be used pulverized product and/or extract of *Angelica keiskei* koidz. containing in a high content a polyphenol, which is a component derived from *Angelica keiskei* koidz., the polyphenol having enhancing activity for NGF production, such as caffeic acid methyl ester, caffeic acid ethyl ester, a chalcone compound, a coumarine compound, or a chroman compound and/or a pharmacologically acceptable salt thereof. Here, the chalcone compound derived from *Angelica keiskei* koidz. is exemplified by xanthoangelol; the coumarine compound is exemplified by 7-O-β-D-glucopyranosyloxy-8-prenylcoumarin, 7-β-D-glucopyranosyloxy-6-prenylcoumarin, 3'-O-β-D-glucopyranoyl khellactone and 4'-O-angeloyl-3'-O-[6-O-(β-D-glucopyranosyl)-β-D-glucopyranosyl]-khellactone; and the chroman compound is exemplified by 8-carboxyl-3-hydroxy-5-methoxy-2-dimethylchroman and 3-hydroxy-8-[3-(4-hydroxyphenyl)-acryloyl]-5-methoxy-2-dimethylchroman. The medicament, food, beverage or feed of the present invention containing these components in a high content can be manufactured by using the pulverized product and/or extract of *Angelica keiskei* koidz. containing the above-mentioned components in a high content.

The term "high content" as referred to herein means that the content of one or more of the above-mentioned components (weight) per unit weight of the pulverized product and/or extract *of Angelica keiskei* koidz. [content ratio (% by weight) of one or more of the above-mentioned components in pulverized product and/or extract *of Angelica keiskei* koidz.] is higher than the content of the above-mentioned component (weight) per unit weight of the raw material *Angelica keiskei* koidz. [content ratio (% by weight) of the above-mentioned component in *Angelica keiskei* koidz.], i.e. the above-mentioned component is more condensed in its pulverized product and/or extract than that of the raw material *Angelica keiskei* koidz. Concretely, the high content refers to a content ratio of the above-mentioned component in the pulverized product and/or extract *of Angelica keiskei* koidz. of preferably 1.5-folds or more, more preferably 2-folds or more, even more preferably 3-folds or more that of a content ratio of the above-mentioned component in *Angelica keiskei* koidz. In addition, the term "high content" means that, as concretely expressed by a content ratio of the above-mentioned component in the pulverized product and/or extract of *Angelica* *keiskei* koidz., the above-mentioned component is contained in the pulverized product and/or extract in an amount of preferably 0.00001% by weight or more, more preferably 0.0001% by weight or more. The same can be said for the pharmacologically acceptable salt of the above-mentioned component.

The pulverized product and extract *of Angelica keiskei* koidz. containing the above-mentioned component in a high content, and the pulverized product and extract containing the pharmacologically acceptable salt of the above-mentioned component in a high content can be appropriately obtained in the same manner as that of *Humulus lupulus* described above.

The pharmacologically acceptable salt usable in the present invention is exemplified by, for instance, alkali metal salts, alkaline earth metal salts, salts with an organic base, and the like. The pharmacologically acceptable salt usable in the present invention means a salt of a compound that is substantially nontoxic against an organism, wherein the salt has an enhancing activity for NGF production. The salt includes, for instance, salts with sodium, potassium, calcium, magnesium, ammonium or protonated benzathine (N,N'-di-benzylethylenediamine), choline, ethanolamine, diethanolamine, ethylenediamine, meglamine (N-methylglucamine), benethamine (N-benzylphenetylamine), piperazine or tolomethamine (2-amino-2-hydroxymethyl-1,3-propanediol).

The pulverized product and/or extract obtained from *Humulus lupulus* and/or *Angelica keiskei* koidz., containing the pharmacologically acceptable salt of the component can be prepared by, for instance, pulverization or extraction in the coexistence of the above-mentioned alkali metal salt or the like when the pulverized product or extract of *Humulus lupulus* or *Angelica keiskei* koidz. is obtained by the above process.

Each of the pulverized product or extract of *Humulus lupulus* or *Angelica keiskei* koidz. obtained in the manner as described above can be used as an effective ingredient of the present invention alone or in admixture of two or more kinds.

The effective ingredient of the present invention is not especially found to be toxic as described below. Also, there is no risk of the incidence of adverse actions. Therefore, the enhancement for NGF production can be safely and appropriately carried out. Accordingly, the medicament, food, beverage or feed of the present invention each comprising the effective ingredient is effective for treatment or prevention of a disease requiring enhancement of NGF production for the treatment or prevention.

NGF is an endogenous growth factor for maintaining viability and functions of nerve cells, elongating nerve cells in accordance with a concentration gradient of NGF, or the like. Therefore, by enhancing NGF production by the effective ingredient of the present invention, the treatment or prevention of senile dementia such as Alzheimer's disease, peripheral nerve disorder, cerebrovascular disorder, cerebral tumor, cerebral apicitis, nerve degenerative disease caused by head injury, diseases requiring recovery and regeneration of nerve functions, caused by intoxication with an anesthetic, and the like can be carried out. In addition, it is useful in the treatment or prevention of diabetic peripheral nerve disorder, amyotrophic lateral sclerosis, drug-induced peripheral nerve disorder, Parkinson's disease, Huntington's disease, sensory nerve disorder, retinitis pigmentosa, macular dystrophy, and the like.

The disease requiring the enhancement of NGF production in the present invention is not particularly limited, as long as the disease can be treated or prevented by enhancing NGF production with a therapeutic agent, a prophylactic agent or the like, each comprising the above-mentioned effective ingredient. Among them, the therapeutic agent or prophylactic agent, which is the medicament of the present invention, is especially effective against various diseases exemplified above which can be treated or prevented by enhancing NGF production.

Also, the medicament of the present invention can exhibit an action of ameliorating or improving learning and memorizing ability by its enhancing activity for NGF production. Therefore, the present invention provides an agent for ameliorating or improving learning and memorizing ability. The action for ameliorating or improving learning and memorizing ability of the effective ingredient of the present invention can be evaluated in accordance with, for instance, the method described in Example 6 set forth below.

The therapeutic agent or prophylactic agent of the present invention for a disease requiring enhancement of NGF production includes those formed into a preparation by combining the above-mentioned effective ingredient according to the present invention with a known pharmaceutical carrier.

The therapeutic agent or prophylactic agent is usually manufactured by formulating the above-mentioned effective ingredient with a pharmacologically acceptable liquid or solid carrier suitable for oral administration or intravenous administration. In addition, there may be optionally added thereto a solvent, a dispersant, an emulsifier, a buffer, a stabilizer, an excipient, a binder, a disintegrant, a lubricant, or the like, thereby being usually made into a solid agent such as a tablet, a granule, a powder, a fine powder, and a capsule, or a liquid agent such as a common liquid agent, a suspension agent or an emulsion agent. In addition, there can be also prepared a dry product which can be made liquid by adding an appropriate carrier before use.

By selecting oral administration or intravenous administration for a method for administering the therapeutic agent or prophylactic agent of the present invention, the enhancing action for NGF production owned by the therapeutic agent or prophylactic agent of the present invention can be effectively exhibited. In item (2) of Example 3 of the present specification, the intraperitoneal administration is carried out, and the same effects can be also expected in the intravenous administration.

The pharmaceutical carrier can be selected depending upon the above-mentioned administration form and preparation form of the therapeutic agent or prophylactic agent. In the case of an orally administered preparation as a solid preparation, the preparation can be made into the form of a tablet, a pill, a capsule, a powder, a fine powder, a granule or the like. For instance, starch, lactose, saccharose, mannitol, carboxymethyl cellulose, cornstarch, an inorganic salt or the like can be utilized as a carrier. In addition, during the preparation of the orally administered preparation, a binder, a disintegrant, a surfactant, a lubricant, a fluidity accelerator, a flavor, a colorant, a perfume, or the like can be further formulated. In the case of forming into a tablet or pill, for instance, the tablet or pill may be covered with a sugar-coating made of sucrose, gelatin or hydroxypropyl cellulose, or with a film made of a substance soluble in the stomach or intestine as desired. In the case of an orally administered preparation as a liquid composition, the preparation can be prepared in the form of a pharmacologically acceptable emulsion, solution, suspension, syrup, or the like. In this case, for instance, purified water, ethanol or the like is utilized as a carrier. Furthermore, an auxiliary agent such as a wetting agent or a suspending agent, a sweetener, a flavor, an antiseptic, or the like may be added as desired.

On the other hand, in the case of an intravenously administered preparation, the above-mentioned effective ingredient of the present invention is dissolved or suspended in a diluent such as distilled water for injection, physiological saline, an aqueous solution of glucose, vegetable oil for injection, sesame oil, peanut oil, soybean oil, corn oil, propylene glycol or polyethylene glycol, according to a conventional method, and adding a microbicide, a stabilizer, an osmotic regulator, a soothing agent, or the like as desired. It is also possible to produce a solid composition which can be used by dissolving the solid preparation in sterile water or a sterile solvent for injection before use.

Each of the above-mentioned various preparations can be appropriately produced by conventional methods by utilizing known pharmaceutical carriers and the like. Also, the content of the effective ingredient in the preparation is not particularly limited, as long as the content is in an amount so that the effective ingredient can be administered preferably within the dose described below in consideration of administration form or the like of the preparation. In general, the content of the effective ingredient in the medicament of the present invention, in terms of the amount of xanthohumol derived from *Humulus lupulus* and/or a pharmacologically acceptable salt thereof, is preferably 0.05% by weight or more, more preferably 0.1 % by weight or more. In addition, the content of the effective ingredient, in terms of the amount of the above-mentioned polyphenol derived from *Angelica keiskei* koidz. and/or a pharmacologically acceptable salt thereof, is preferably 0.00001% by weight or more, more preferably 0.0001% by weight or more. The useful effects for the therapeutic agent and prophylactic agent of the present invention can be obtained by orally administering and/or intravenously injecting the agent.

The dose for the therapeutic agent or prophylactic agent of the present invention is changeable and properly set depending upon its preparation form, administration method, purpose of use, age, body weight, symptom or the like of the patient to which the therapeutic agent or prophylactic agent is applied, or the like. In general, the dose for the agent, in terms of the amount of the above-mentioned effective ingredient contained in the preparation, is preferably from 0.1 µg to 100 g/kg, more preferably from 0.01 mg to 10 g/kg, per day for adult. In addition, in a case where xanthohumol and/or a pharmacologically acceptable salt thereof is used as an effective ingredient, the dose for the agent, in terms of the amount of the effective ingredient, is preferably from 0.01 to 1000 mg/kg, especially preferably from 0.1 to 100 mg/kg per day for adult.

In the present invention, according to the use embodiment of a conventional general orally administered preparation or intravenously injection preparation, xanthohumol and/or a pharmacologically acceptable salt thereof can be administered in a high dose of 0.1 mg/kg or more per day for adult. At this time, there can be preferably used a therapeutic agent or prophylactic agent comprising as an effective ingredient a pulverized product and/or extract derived from *Humulus lupulus,* in which xanthohumol and/or a pharmacologically acceptable salt thereof is contained in a high content; or a therapeutic agent or prophylactic agent comprising as an effective ingredient xanthohumol and/or a pharmacologically acceptable salt thereof. In addition, when the pulverized product and/or extract derived from *Angelica keiskei* koidz. is used as an effective ingredient, the dose of the pulverized product and/or extract is preferably from 0.01 to 100 g/kg, especially preferably from 0.1 to 10 g/kg per day for adult. In the present invention, according to the use embodiment of a conventional general orally administered preparation or intravenously injection preparation, the above-mentioned component derived from *Angelica keiskei* koidz. and/or a pharmacologically acceptable salt thereof can be administered in a high dose of 0.1 mg/kg or more per day for adult. At this time, there can be preferably used a therapeutic agent or prophylactic agent comprising as an effective ingredient a pulverized product and/or extract derived from *Angelica keiskei* koidz., in which the above-mentioned component derived from *Angelica keiskei* koidz. and/or a pharmacologically acceptable salt thereof is contained in a high content; or a therapeutic agent or prophylactic agent comprising as an effective ingredient the above-mentioned component derived from *Angelica keiskei* koidz. and/or a pharmacologically acceptable salt thereof.

The dose varies depending upon various conditions, so that an amount smaller than the dose mentioned above may be sufficient, or an amount exceeding the dose range may be required. Administration period is not limited. Administration may be carried out once or several divided portions in a day within the desired dose range. Also, the therapeutic or prophylactic agent of the present invention can not only be orally administered or intravenously administered *per se* but also be taken on a daily basis by adding the agent to optional foodstuff.

In addition, the present invention can provide an enhancing agent for NGF production comprising the above-mentioned effective ingredient. The enhancing agent may be the above-mentioned effective ingredient itself, or a composition comprising the above-mentioned effective ingredient. In the embodiment of the present invention, the salt used as the effective ingredient is preferably a pharmacologically acceptable salt. The enhancing agent for NGF production may be produced by, for instance, formulating the above-mentioned effective ingredient with other ingredients which can be used for the same application as the effective ingredient, and forming into a form of reagent usually used according to the above-mentioned process for manufacturing the therapeutic agent or prophylactic agent. The content of the above-mentioned effective ingredient in the enhancing agent is not particularly limited, as long as the content is in an amount so that the desired effects of the present invention can be exhibited in consideration of administration form, use purpose or the like of the enhancing agent. In general, the content of the effective ingredient in the enhancing agent for NGF production of the present invention, in terms of the amount of xanthohumol derived from *Humulus lupulus* and/or a pharmacologically acceptable salt thereof, is in an amount of preferably 0.05% by weight or more, more preferably 0.1% by weight or more. In addition, the content of the effective ingredient in the enhancing agent, in terms of the above-mentioned polyphenol derived from *Angelica keiskei* koidz. and/or a pharmacologically acceptable salt thereof, is preferably 0.00001% by weight or more, more preferably 0.0001% by weight or more. In addition, the amount of the enhancing agent used is not particularly limited as long as the desired effects of the present invention can be exhibited. The enhancing agent may be used, for instance, in an amount so that the effective ingredient can be administered within the dose range of the effective ingredient in the above-mentioned therapeutic agent or prophylactic agent. The enhancing agent is useful for enhancement of NGF production in a disease requiring enhancement for NGF production. In addition, the enhancing agent is also useful for screening of drugs for NGF-associated diseases. Further, the enhancing agent is useful for functional studies of NGF or physical changes of nerve cells.

In addition, the present invention provides a food, beverage or feed for enhancing NGF production, in which the above-mentioned effective ingredient is contained, added and/or diluted. In the embodiment of the present invention, a pharmacologically acceptable salt, or a salt having the same level of safety may be used as the salt of the effective ingredient. The food, beverage or feed of the present invention is very useful for ameliorating or preventing symptoms of a disease requiring enhancement of NGF production by its enhancing action of NGF production. Also, the food, beverage or feed of the present invention is very useful for obtaining an effect of ameliorating or improving learning and memorizing ability. Therefore, the present invention provides a food, beverage or feed for ameliorating or improving learning and memorizing ability.

The process for preparing the food, beverage or feed of the present invention is not particularly limited. For instance, formulation, cooking, processing, and the like can be carried out in accordance with those generally employed for foods, beverages or feeds, and the food, beverage or feed can be prepared by the general methods for preparing a food, beverage or feed, as long as the resulting food, beverage or feed contain the above-mentioned effective ingredient according to the present invention, which has an enhancing action for NGF production.

The food or beverage of the present invention is not particularly limited. The food or beverage includes, for instance, processed agricultural and forest products, processed stock raising products, processed marine products and the like, including processed grain products such as processed wheat products, processed starch products, processed premix products, noodles, macaronis, bread, bean jam, buckwheat noodles, wheat-gluten bread, rice noodle, *fen-tiao*, and packed rice cake; processed fat and oil products such as plastic fat and oil, tempura oil, salad oil, mayonnaise, and dressing; processed soybean products such as tofu products, soybean paste, and fermented soybeans; processed meat products such as ham, bacon, pressed ham, and sausage; marine products such as frozen ground fish, boiled fish paste, tubular roll of boiled fish paste, cake of ground fish, deep-fried patty of fish paste, fish ball, sinew, fish meat ham and sausage, dried bonito, products of processed fish egg, marine cans, and preserved food boiled down in soy sauce (*tsukudani*); milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chocolates, biscuits, sweet bun, cake, rice cake snacks and rice snacks; alcohol beverages such as *sake,* Chinese liquor, wine, whiskey, Japanese distilled liquor (*shochu*), vodka, brandy, gin, rum, beer, refreshing alcoholic beverages, fruit liquor, and liqueur; luxury drinks such as green tea, tea, oolong tea, coffee, refreshing beverages and lactic acid beverages; seasonings such as soy sauce, sauce, vinegar, and sweet rice wine; canned, binned or pouched foods such as rice topped cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste *(miso)* soup, precooked foods, precooked beverages, and precooked soup; frozen foods such as *sukiyaki*, pot-steamed hotchpotch, split and grilled eel, hamburger steak, *shao-mai*, dumpling stuffed with minced pork, various sticks, and fruit cocktails; solid foods; liquid foods (soups); spices; and the like, each comprising the above-mentioned effective ingredient according to the present invention.

The food or beverage of the present invention does not have any particular limitation on its shape, as long as the above-mentioned effective ingredient is contained, added and/or diluted, singly or in plurality, in an amount necessary for exhibiting its enhancing action for NGF production. The shapes also include orally taken shapes such as tablets, granules and capsules.

In addition, as to the beverage of the present invention, there can be prepared into healthcare drink by mixing the effective ingredient of the present with a squeezed juice of a plant other than those belonging to Umbelliferae, for instance, a vegetable, a fruit or the like, or squeezing the plant together with the plant belonging to Umbelliferae. For instance, the healthcare drink for enhancing NGF production can be prepared by diluting a squeezed juice of *Angelica keiskei* koidz. with water, or mixing the squeezed juice with a squeezed juice of *Daucus, Brassica Rapa* var. *pervidis* (*komatsuna*), Japanese turnip, Qing gin cai, tomato, mandarin orange, lemon, grapefruit, kiwi, spinach, radish, Japanese radish (*daikon*), Chinese cabbage, cabbage, sunny lettuce, lettuce, *Allium odorum,* okra, green pepper, cucumber, kidney beans, green soybeans, pea, Indian corn, garlic, Rocket, loquat, *Citrus natsudaidai, amanatsu*, or the like. In addition, the healthcare drink for enhancing NGF production can be obtained by mixing the effective ingredient of the present invention with cow's milk, soybean milk or the like.

The content of the above-mentioned effective ingredient in the food or beverage of the present invention is not particularly limited, and the content can be appropriately selected from the viewpoints of sensory aspect and exhibition of activity. The content of the effective ingredient in the food is, for instance, preferably 0.00001% by weight or more, more preferably from 0.0001 to 100% by weight, even more preferably from 0.0006 to 90% by weight. The content in the beverage is, for instance, preferably 0.00001% by weight or more, more preferably from 0.0001 to 100% by weight, even more preferably from 0.0006 to 90% by weight. In general, the content of the effective ingredient in the food or beverage of the present invention, in terms of the amount of xanthohumol derived from *Humulus lupulus* and/or a pharmacologically acceptable salt thereof, is in an amount of preferably 0.05% by weight or more, more preferably 0.1% by weight or more. In addition, the content of the effective ingredient in the food or beverage, in terms of the above-mentioned polyphenol derived from *Angelica keiskei* koidz. and/or a pharmacologically acceptable salt thereof, is preferably 0.00001% by weight or more, more preferably 0.0001% by weight or more. Also, the food or beverage of the present invention may be taken so that the effective ingredient contained therein is taken in an amount of preferably from 0.001 mg to 100 g/kg, more preferably from 0.01 mg to 10 g/kg, per day for adult. In addition, in a case where xanthohumol and/or a pharmacologically acceptable salt thereof is used as an effective ingredient, for instance, the food or beverage is taken so that the effective ingredient is taken in an amount of preferably from 0.01 to 1000 mg/kg, especially preferably from 0.1 to 100 mg/kg per day for adult. According to the present invention, there is provided a food or beverage containing xanthohumol and/or a pharmacologically acceptable salt thereof in a high content, by which xanthohumol and/or a pharmacologically acceptable salt thereof can be taken in an amount of 0.1 mg/kg or more per day for adult according to the use embodiment of a conventional general food or beverage. In addition, in a case where a pulverized product and/or extract of *Angelica keiskei* koidz. is used as an effective ingredient, the food or beverage may be taken so that the effective ingredient is taken in an amount of preferably from 0.01 to 100 g/kg, especially preferably from 0.1 to 10 g/kg, per day for adult. According to the present invention, there is provided a food or beverage containing the above component derived from *Angelica keiskei* koidz. and/or a pharmacologically acceptable salt thereof in a high content, by which the above component derived from *Angelica keiskei* koidz. and/or a pharmacologically acceptable salt thereof can be taken in an amount of 0.1 g/kg or more per day for adult according to the use embodiment of a conventional general food or beverage.

In addition, the present invention provides a feed for an organism having enhancing action for NGF production, prepared by containing, adding and/or diluting the above-mentioned effective ingredient. In still another embodiment, the present invention also provides a method of feeding an organism, characterized by administering the above-mentioned effective ingredient to the organism. In still yet another embodiment, the present invention provides an organism feeding agent characterized in that the organism feeding agent comprises the above-mentioned effective ingredient.

In these inventions, the organisms are, for instance, culturing or breeding animals, pet animals, and the like. The culturing or breeding animal is exemplified by cattle, laboratory animals, poultry, pisces, crustaceae or shellfish. The feed is exemplified by a feed for sustenance of and/or amelioration in physical conditioning. The organism feeding agent is exemplified by immersion agents, feed additives, and beverage additives.

According to these inventions, the same effects can be expected to be exhibited as those of the above-mentioned therapeutic agent or prophylactic agent of the present invention, on the basis of the enhancing action for NGF production of the above-mentioned effective ingredient usable in the present invention, in the organism exemplified above for applying these. In other words, the feed or the like of the present invention has a therapeutic or prophylactic effect for a disease requiring enhancing action for NGF production in the organism. In addition, the feed of the present invention has an effect of ameliorating or improving learning and memorizing ability of the above-mentioned organism. Therefore, the present invention also provides a feed for ameliorating or improving learning and memorizing ability.

The above-mentioned effective ingredient usable in the present invention is usually administered in an amount of preferably from 0.001 mg to 100 g, especially preferably from 0.01 mg to 10 g, per day per 1 kg of the body weight of the subject organism. In addition, in a case where xanthohumol and/or a pharmacologically acceptable salt thereof is used as its effective ingredient, the feed may be taken so that the effective ingredient is administered in an amount of preferably from 0.01 to 1000 mg, especially preferably from 0.1 to 100 mg, per day per 1 kg of the body weight of the subject organism. According to the present invention, there is provided a feed containing xanthohumol and/or a pharmacologically acceptable salt thereof in a high content, by which xanthohumol and/or a pharmacologically acceptable salt thereof can be taken in an amount of 0.1 mg/kg or more per day per 1 kg of the body weight of the subject organism according to the use embodiment of a conventional general feed. In addition, in a case where a pulverized product and/or extract of *Angelica keiskei* koidz. is used as an effective ingredient, the feed may be taken so that the effective ingredient is administered in an amount of preferably from 0.01 to 100 g/kg, especially preferably from 0.1 to 10 g/kg, per day per 1 kg of the body weight of the subject organism. According to the present invention, there is provided a feed containing the above component derived from *Angelica keiskei* koidz. and/or a pharmacologically acceptable salt thereof in a high content, by which the above component derived from *Angelica keiskei* koidz. and/or a pharmacologically acceptable salt thereof can be taken in an amount of 0.1 g/kg or more per day per 1 kg of the body weight of the subject organism according to the use embodiment of a conventional general feed.

The administration can be made using the feed of the present invention prepared by adding and mixing the effective ingredient of the present invention in a raw material for an artificially formulated feed to be given to a subject organism, or using a feed prepared by mixing the effective ingredient of the present invention with a powder raw material for an artificially formulated feed to give an organism feeding agent, and thereafter further adding and mixing the agent with other raw materials. The content of the above-mentioned effective ingredient in the feed is not particularly limited. The content can be appropriately set in accordance with its purposes, and the content is in a ratio of preferably from 0.001 to 90% by weight. In general, the content of the effective ingredient in the feed of the present invention, in terms of the amount of xanthohumol derived from *Humulus lupulus* and/or a pharmacologically acceptable salt thereof, is in an amount of preferably 0.05% by weight or more, more preferably 0.1% by weight or more. In addition, the content of the effective ingredient in the feed, in terms of the above-mentioned polyphenol derived from *Angelica keiskei* koidz. and/or a pharmacologically acceptable salt thereof, is preferably 0.00001% by weight or more, more preferably 0.0001% by weight or more.

The artificially formulated feed includes feeds using animal-derived raw materials such as fish meal, casein, and squid meal; plant-derived raw materials such as soybean grounds, flour, and starch; microorganism raw materials such as yeasts for feed; animal fats and oils such as cod-liver oil and squid-liver oil; vegetable fats and oils such as soybean oil and rapeseed oil; and vitamins, minerals, amino acids, and antioxidants; and the like as raw materials. In addition, feeds for fish such as fish minced meat are also included.

The process for preparing the feed according to the present invention is not particularly limited, and its composition may be set in accordance with a general feed, as long as the above-mentioned effective ingredient according to the present invention having enhancing action for NGF production may be contained in the feed prepared.

Also, the effective ingredient according to the present invention having enhancing action for NGF production can be administered by directly adding the effective ingredient to water, seawater, or the like in a pool, a water tank, a water reservoir, or a feeding range, and immersing a subject organism into the resulting solution. The immersion method is especially effective when the amount of intake of the feed of the subject organism is lowered. The concentration of the above-mentioned effective ingredient according to the present invention having enhancing action for NGF production in water or seawater is not particularly limited, and the concentration may be set in accordance with its purposes. It is appropriate that the concentration is in a ratio of preferably from 0.00001 to 20% by weight.

Also, a beverage comprising the above-mentioned effective ingredient according to the present invention having enhancing action for NGF production may be given to a subject organism as a feeding drink. The concentration of the effective ingredient used in the present invention having enhancing action for NGF production in the beverage is not particularly limited, and the concentration may be set in accordance with its purposes. It is appropriate that the concentration is preferably in a ratio of from 0.0001 to 90% by weight. The organism feeding agent, for instance, an immersion agent, a feed additive, or a beverage additive, comprising the above-mentioned effective ingredient according to the present invention having enhancing action for NGF production may be prepared by a known formulation and preparation process. The content of the effective ingredient in the organism feeding agent is not particularly limited, so long as the desired effects of the present invention can be obtained.

The organism to which the present invention can be applied is not limited. The culturing or breeding animals include cattle such as *Equus, Bos, Porcus, Ovis, Capra, Camelus,* and *Lama;* experimental animals such as mice, rats, guinea pigs, and rabbits; poultry such as *Chrysolophus*, ducks, *Meleagris,* and *Struthioniformes;* pisces such as *Pagrus, Oplegnathidae, Paralichthys,* plaice, *Seriola,* young *Seriola,* amberjack, *Thunna, Caranx delicatissimus, Plecoglossus,* *Salmo•Oncorhynchus, Fugu, Anguilla, Misguirus,* and *Parasilurus;* Crustaceae such as *Penaidae,* black tiger shrimp, *Penaeus roentalis,* and *Portulus trituberculatus*; and shellfish such as abalones (*awabi*), turban shells, scallops, and oysters; and the pet animals includes dogs, cats, and the like, so that the feed can be widely applied to animals on land and in water.

By allowing a subject organism to take the feed comprising the above-mentioned effective ingredient usable in the present invention having enhancing action for NGF production, or immersing a subject organism into a solution containing the above-mentioned effective ingredient usable in the present invention having enhancing action for NGF production, the physical conditions of the cattle, experimental animals, poultry, pisces, Caustacea, shellfish, pet animals or the like can be well sustained and ameliorated. In addition, the learning and memorizing ability of the subject organism can be ameliorated or improved.

As a still another embodiment of the present invention, there is provided use of the above-mentioned effective ingredient according to the present invention in the preparation of a therapeutic agent or prophylactic agent for a disease requiring enhancement of NGF production, an enhancing agent for NGF production, or a food, beverage or feed for enhancing NGF production. The use embodiments include each of the use embodiments of the above-mentioned effective ingredient in the preparation of the therapeutic agent or prophylactic agent, the enhancing agent for NGF production, or the food, beverage or feed for enhancing NGF production of the present invention mentioned above. For instance, as the use of the above-mentioned effective ingredient in the preparation of a therapeutic agent or prophylactic agent for a disease requiring enhancement of NGF production, or an enhancing agent for NGF production, there are exemplified the use in the preparation of a solid agent such as a tablet, a granule, a powder, a fine powder, and a capsule, a liquid agent such as a common liquid agent, a suspension agent, or an emulsion agent, or a dry product which can be liquefied by adding an appropriate carrier before use.

Further, in a still another embodiment of the present invention, there can be provided a method for enhancing NGF production, comprising administering the above-mentioned effective ingredient according to the present invention to an animal. This method can be carried out by administering the above-mentioned effective ingredient, preferably as the above-mentioned enhancing agent for NGF production, to an animal that is predicted to require or requires enhancement of NGF production, whereby NGF production is enhanced. The administration method, dose, or the like of the effective ingredient may be the same as those of the above-mentioned enhancing agent for NGF production. In the method for enhancing NGF production, the therapeutic agent or prophylactic agent, or the food, beverage or feed of the present invention can be also used. In addition, the term "animal" includes, for instance, a mammal such as human, dogs, cats, *Bos, Porcus, Equus,* and the like, among which the method is preferably used for human. The method for enhancing NGF production is useful for, for instance, the enhancement of NGF production in a case of treatment or prevention of a disease requiring enhancement for NGF production, or amelioration or improvement of learning and memorizing ability of an organism. In addition, the method is also useful for screening of drugs for diseases associated with NGF. Furthermore, the method is useful for functional studies concerning NGF or physical changes in nerve cells.

No toxicity is found even when the above-mentioned effective ingredient usable in the present invention is administered in an amount effective for the exhibition of its action. For instance, there are no incidences of adverse actions even when any one of a pulverized product of *Humulus lupulus,* an extract from *Humulus lupulus,* xanthohumol, a pulverized product *of Angelica keiskei* koidz. and an extract from *Angelica keiskei* koidz. is administered to a mouse at 1 g/kg in a single dose, and no cases of deaths are found.

### EXAMPLES

The present invention will be more concretely described hereinbelow by means of the examples, without by no means limiting the scope of the present invention thereto. Unless specified otherwise, "%" in the examples means "% by weight."

### Example 1

(1) Five-hundred liters of ethanol was added to 50 kg of a commercially available dried *Humulus lupulus (Humulus lupulus* CSA made in Czech Republic, purchased from Mitsubishi Corporation). The mixture was stirred at room temperature for 2 hours, and thereafter filtered to give an extract from *Humulus lupulus.* The extract from *Humulus lupulus* was concentrated under a reduced pressure, to give 30 liters of a concentrate.
(2) The above-mentioned concentrate was subjected to a liquid-liquid partition treatment with hexane and a 85% aqueous ethanol. A layer of 85% aqueous ethanol was concentrated under a reduced pressure, to give 3.5 liters of a concentrate. The concentrate was dissolved in an equivolume of a mixture of chloroform : hexane (5:2), and thereafter the resulting mixture was subjected to chromatography using a silica gel column (manufactured by Fuji Silycia Kagaku K.K.; BW-300SP, amount of resin 7.0 liters). The silica adsorbent was washed with 10 liters of hexane : chloroform (1:1), and eluted with 12 liters of chloroform : ethyl acetate (8:2). The eluate was concentrated under a reduced pressure, and a procedure of recrystallization from hexane and ethyl acetate against 700 milliliters of the obtained concentrate was repeated three times, to give 37.5 g of xanthohumol.

### Example 2

Ten kilograms of root portions of *Angelica keiskei* koidz. were washed, and cut into pieces of 3 mm widths. The root portions were sterilized by heating them in 200 liters of hot water at 95°C for 45 seconds. The obtained cut pieces *of Angelica keiskei* koidz. were quickly frozen at -35°C for 2 to 3 hours, and thereafter vacuum-dried at 0.5 atm for 24 hours. Two kilograms of the obtained cut pieces *of Angelica keiskei* koidz. were pulverized with a pulverizer at 120 mesh or more, to give 2 kg of a pulverized product of *Angelica keiskei* koidz.

### Example 3

(1) Mice (ddY-type, 7-week old male) and rats (SD-type, 7-week old male) were purchased from Japan SLC, Inc., and an acute toxicity test by a single administration of xanthohumol described in Example 1 was carried out. Xanthohumol described in Example 1 was suspended and diluted in distilled water, and the suspension was administrated orally or intraperitoneally at 10, 100 and 1000 mg/kg body weight. Three cases for each group were observed up to 48 hours after the administration. As a result, no cases of deaths were found in either group.
(2) Male SD rats were purchased from Japan SLC, Inc., and used for an experiment from 7-week old after preliminary rearing. Xanthohumol described in Example 1 was suspended in distilled water, and the rats were subjected to forced oral administration of the suspension at a ratio of 3 and 10 mg of xanthohumol respectively per 1 kg of body weight. In addition, the suspension was intraperitoneally administered at a ratio of 1, 3 or 10 mg of xanthohumol. A control group was administered with distilled water in the same manner as above. In either group, four rats were administered once a day for consecutive days. In the case of the forced oral administration, the submandibular gland and the brain were excised on the fifth day from the beginning of administration. In the case of the intraperitoneal administration, ischiadic nerve and gastrocneminal muscle were excised at 4 hours after the end of administration on the third day. The excised tissues were homogenized and centrifuged, and the NGF contents in the supernatant were determined by enzyme immunoassay method (manufactured by Roche).

The results are shown in Tables 1 to 4. The numbers in the tables represent an average value ± standard error for the four cases. In addition, an asterisk * symbol means that the group has a significant difference at a significance level of 5% or less as compared to the control group according to Student-t test, and a triple asterisk *** symbol means that the group similarly has a significant difference at a significance level of 0.1% or less.

**Table 1**

| | NGF Content in Submandibular Gland of Rat (ng/g tissue) |
|---|---|
| Group Orally Administered with 10 mg/kg Xanthohumol | 13.39 ± 0.65*** |
| Group Orally Administered with 3 mg/kg Xanthohumol | 8.88±0.98*** |
| Control Group | 0.11±0.01 |
| Average Value ± Standard Error | |

**Table 2**

| | NGF Content in Brain of Rat (pg/g tissue) |
|---|---|
| Group Orally Administered with 10 mg/kg Xanthohumol | 524.9 ± 200.7 |
| Group Orally Administered with 3 mg/kg Xanthohumol | 508.2 ± 95.1 |
| Control Group | 401.3 ± 158.5 |
| Average Value ± Standard Error | |

**Table 3**

| | NGF Content in Ischiadic Nerve of Rat (pg/g tissue) |
|---|---|
| Group Intraperitoneally Administered with 10 mg/kg Xanthohumol | 445.7 ± 59.1 |
| Group Intraperitoneally Administered with 3 mg/kg Xanthohumol | 351.6 ± 26.8 |
| Group Intraperitoneally Administered with 1 mg/kg Xanthohumol | 386.4 ± 30.1 * |
| Control Group | 302.9 ± 7.5 |
| Average Value ± Standard Error | |

**Table 4**

| | NGF Content in Gastrocneminal Muscle of Rat (pg/g tissue) |
|---|---|
| Group Intraperitoneally Administered with 10 mg/kg Xanthohumol | 56.8 ± 3.3 |
| Group Intraperitoneally Administered with 3 mg/kg Xanthohumol | 53.9 ± 4.0 |
| Group Intraperitoneally Administered with 1 mg/kg Xanthohumol | 48.0 ± 3.4 |
| Control Group | 45.7 ± 5.4 |
| Average Value ± Standard Error | |

As compared to each of the control groups, the groups administered with xanthohumol showed an increase in the NGF content in each of the tissues. The action of xanthohumol was especially remarkable in the submandibular gland. The submandibular gland has been reported to be one of the NGF-producing tissues in a living body, and it is considered that the administered xanthohumol accelerated the NGF production in a living body, thereby increasing their contents. Also, from the findings that the NGF contents were increased in the brain and in the gastrocneminal muscle, which is a controlling muscle of peripheral nerve and ischiadic nerve, it was shown that the xanthohumol also enhanced NGF production in the central nervous system and the peripheral nervous system. In addition, from the findings that these effects were obtained in the intraperitoneal administration, the similar effects can be also expected in the intravenous administration.

### Example 4

Male SD rats were purchased from Japan SLC, Inc., and used for an experiment from 7-week old after preliminary rearing. The powder *of Angelica keiskei* koidz. described in Example 2 was mixed with a powder feed at a ratio of 1%, and the rats were allowed to take the mixture *ad libitum*. The control group was given only the powder feed. Four rats were used for either group, and the gastrocneminal muscle was excised on the fifth day from the beginning of administration. The excised tissues were homogenized and centrifuged, and the NGF contents of the supernatant thereof were determined by enzyme immunoassay method (manufactured by Roche).

The results are shown in Table 5. The numbers in the table represent an average value ± standard error for the four cases. In addition, an asterisk * symbol means that the group has a significant difference at a significance level of 5% or less as compared to the control group according to Student-t test.

**Table 5**

| | NGF Content in Gastrocneminal Muscle of Rat (pg/g tissue) |
|---|---|
| Group Administered with 1% *Angelica keiskei* koidz. | 83.2 ± 3.2* |
| Control Group | 71.2 ± 3.1 |
| Average Value ± Standard Error | |

As compared to the control group, the group administered with *Angelica keiskei* koidz. showed an increase in the NGF content in the gastrocneminal muscle. Since the gastrocneminal muscle is a controlling muscle of peripheral nerve, it was shown that the *Angelica keiskei* koidz. enhanced the NGF production in the peripheral nervous system. In addition, an effective dose of the powder *of Angelica keiskei* koidz. was calculated from an amount of intake of the powder feed, and the effective dose was found to be 0.75 g/kg/day.

### Example 5

Male SD rats were purchased from Japan SLC, Inc., and used for an experiment from 6-week old after preliminary rearing. Streptozotocin (manufactured by nacalai tesque) was intraperitoneally administered in an amount of 60 mg per 1 kg body weight to cause diabetes mellitus. The rats were subjected to grouping in accordance with the blood sugar level on the fifth day from the administration of streptozotocin, and the administration was started. The pulverized product *of Angelica keiskei* koidz. described in Example 2 was mixed with a powder feed at a ratio of 1%, and the rats were allowed to take the mixture *ad libitum*. The control group for diabetes mellitus was only given the powder feed. Also, a group not administered with streptozotocin was referred to as a control group, and given only the powder feed. Four to six rats for each group were continued to be administered, and the ischiadic nerve was excised on the fourth week from the beginning of administration. The excised tissues were homogenized and centrifuged, and the NGF contents in the supernatant was determined by enzyme immunoassay method (manufactured by Roche). The results are shown in Table 6. The numbers in the table represent an average value ± standard error for the four to six cases.

**Table 6**

| Group Administered | NGF Content in Ischiadic Nerve of Rat (pg/g tissue) |
|---|---|
| Control Group for Diabetes Mellitus | 496.9 ± 41.4 |
| Group Administered with 1 % *Angelica keiskei* koidz. | 627.8 ± 51.5 |
| Control Group | 839.5 ± 135.6 |
| Average Value ± Standard Error | |

As compared to the control group, the control group for diabetes mellitus showed a decrease in the NGF content in ischiadic nerve, because a disorder in peripheral nerve occurs along with continuous high blood sugar level. By contrast, the group administered with *Angelica keiskei* koidz. showed an increase in the NGF content in the nerve relative too the control group for diabetes mellitus. From the results, it is considered that the NGF production is enhanced in the peripheral nervous system and the degree of diabetic peripheral nerve disorder is alleviated by the administration of the pulverized product *of Angelica keiskei* koidz. In addition, an effective dose of the pulverized product of *Angelica keiskei* koidz. was calculated from an amount of intake of the powder feed, and the effective dose was found to be 1.6 g/kg/day.

### Example 6

Male Wistar rats were purchased from Japan SLC, Inc., and used for an experiment from 7-week old after preliminary rearing. Ibotenic acid was administered intracerebrally to cause a learning and memory disorder. Nine rats were used for each group, and administration was begun one week after the administration of ibotenic acid. Xanthohumol of Example 1 was suspended in distilled water, and the rats were subjected to forced oral administration with the suspension at a ratio of 10 mg of xanthohumol per 1 kg of body weight. A control group was administered with distilled water in the same manner as above. The rats were administered once a day for consecutive days. A water maze test was carried out on and after the seventh day from the beginning of the administration to evaluate ability of recognizing spaces and directions.

The water maze test method was carried out as follows. Water of about 22°C was charged in a large-scaled water vessel. A platform was set at a height of 2 cm below the water level, and rats were made to enter into water. The rats in water swim around so as to escape from water and take refuge in the platform. The time period from entering into water to taking refuge in the platform was recorded. After taking the refuge, the rats were made to observe surrounding environments on the platform. The maze trial was carried out once, and thereafter the second maze trial was carried out on the next day. The results are shown in Table 7.

**Table 7**

| Group Administered | Time Period (sec) | |
|---|---|---|
| | First Trial | Second Trial |
| Control Group | 133.7 ± 17.5 | 133.4 ± 10.0 |
| Group Administered with Xanthohumol | 140.7 ± 14.7 | 71.3 ± 10.8 |
| Average Value ± Standard Error | | |

The rats learn and memorize the position of platform in association with the surrounding environments by repeating the trial at a specific condition. From Table 7, the time period for taking refuge was shortened in the group administered with xanthohumol as compared to that of the control group. In other words, it was shown that the ability for recognizing spaces and directions was enhanced in the group administered with xanthohumol by the protective action for brain disorders.

### Example 7

A tablet (200 mg/tablet) comprising a pulverized product mainly obtained from leaves and roots of *Angelica keiskei* koidz. as an effective ingredient was prepared on the basis of the formulation of Table 8 in accordance with a conventional method using a tableting machine at a tableting pressure of 3000 kg/cm².

**Table 8**

| Formulation (per Tablet) | |
|---|---|
| Powder of Leaves of *Angelica keiskei* koidz. (mg) | 50 |
| Powder of Roots of *Angelica keiskei* koidz. (mg) | 35 |
| Crystalline Cellulose (mg) | 76 |
| Starch (mg) | 34 |
| Sucrose Fatty Acid Ester (mg) | 4 |
| Calcium Carbonate (mg) | 1 |
| Total | 200 |

### Example 8

A tablet (200 mg/tablet) comprising a pulverized product mainly obtained from flowers of *Humulus lupulus* as an effective ingredient was prepared on the basis of the formulation of Table 9 in accordance with a conventional method using a tableting machine at a tableting pressure of 3000 kg/cm².

**Table 9**

| Formulation (per Tablet) | |
|---|---|
| Powder of *Humulus lupulus* (mg) | 170 |
| Crystalline Cellulose (mg) | 18 |
| Starch (mg) | 2 |
| Sucrose Fatty Acid Ester (mg) | 10 |
| Total | 200 |

### Example 9

A triangular tablet (200 mg/tablet) comprising a pulverized product mainly obtained from leaves and roots of *Angelica keiskei* koidz. as an effective ingredient was prepared on the basis of the formulation of Table 10 in accordance with a conventional method using a tableting machine at a tableting pressure of 3000 kg/cm². The tablet had well-balanced flavor of leaves and roots of *Angelica keiskei* koidz., giving a deep flavor.

**Table 10**

| Formulation (per Tablet) | |
|---|---|
| Powder of Leaves of *Angelica keiskei* koidz. (mg) | 33 |
| Powder of Roots of *Angelica keiskei* koidz. (mg) | 22 |
| Crystalline Cellulose (mg) | 55 |
| Reducing Maltose (mg) | 51 |
| Starch (mg) | 34 |
| Sucrose Fatty Acid Ester (mg) | 4 |
| Calcium Carbonate (mg) | 1 |
| Total | 200 |

### Example 10

A triangular tablet (200 mg/tablet) comprising a pulverized product mainly obtained from *Humulus lupulus* as an effective ingredient was prepared on the basis of the formulation of Table 11 in accordance with a conventional method using a tableting machine at a tableting pressure of 3000 kg/cm². The flavor of the tablet was made to have both a bitter taste of *Humulus lupulus* and a refreshing flavor of a spice (peppermint).

**Table 11**

| Formulation (per Tablet) | |
|---|---|
| Powder of *Humulus lupulus* (mg) | 120 |
| Crystalline Cellulose (mg) | 10 |
| Sugar (mg) | 45 |
| Reducing Maltose (mg) | 10 |
| Starch (mg) | 1 |
| Spice (Peppermint) (mg) | 4 |
| Sucrose Fatty Acid Ester (mg) | 10 |
| Total | 200 |

### INDUSTRIAL APPLICABILITY

According to the present invention, there is provided a medicament for oral administration or intravenous administration that is effective for a disease requiring enhancement of nerve growth factor production, comprising as an effective ingredient a pulverized product and/or extract obtained from *Humulus lupulus* and/or *Angelica keiskei* koidz., or xanthohumol and/or a pharmacologically acceptable salt thereof. The medicament is useful as a therapeutic agent or prophylactic agent for a disease requiring enhancement of nerve growth factor production, such as dementia or a nerve disorder.

Also, according to the present invention, there are provided an enhancing agent for nerve growth factor production, and a food, beverage or feed for enhancing nerve growth factor production, each comprising the above effective ingredient. By taking the food or beverage as foodstuff on a daily basis, the symptoms of a disease requiring enhancement of nerve growth factor production can be ameliorated or the like. In addition, the feed is useful for maintaining homeostasis of a living body by its action of enhancing nerve growth factor production.

Furthermore, these medicament and the like of the present invention are effective for improving or ameliorating learning and memorizing ability of an individual organism.

## Claims

1. A therapeutic agent or prophylactic agent for oral administration or intravenous administration for a disease requiring enhancement of nerve growth factor production for treatment or prevention, **characterized in that** the therapeutic agent or prophylactic agent comprises as an effective ingredient a pulverized product and/or extract obtained from *Humulus lupulus* and/or *Angelica keiskei* koidz.

2. The therapeutic agent or prophylactic agent according to claim 1, **characterized in that** the pulverized product and/or extract obtained from *Humulus lupulus* comprises xanthohumol and/or a pharmacologically acceptable salt thereof in a high content.

3. A therapeutic agent or prophylactic agent for oral administration or intravenous administration for a disease requiring enhancement of nerve growth factor production for treatment or prevention, **characterized in that** the therapeutic agent or prophylactic agent comprises as an effective ingredient xanthohumol and/or a pharmacologically acceptable salt thereof.

4. An enhancing agent for nerve growth factor production for oral administration or intravenous administration, **characterized in that** the enhancing agent comprises as an effective ingredient a pulverized product and/or extract obtained from *Humulus lupulus* and/or *Angelica keiskei* koidz.

5. The enhancing agent according to claim 4, **characterized in that** the pulverized product and/or extract obtained from *Humulus lupulus* comprises xanthohumol and/or a pharmacologically acceptable salt thereof in a high content.

6. An enhancing agent for nerve growth factor production, **characterized in that** the enhancing agent comprises as an effective ingredient xanthohumol and/or a pharmacologically acceptable salt thereof.

7. A food, beverage or feed for enhancing nerve growth factor production, **characterized in that** the food, beverage or feed comprises a pulverized product and/or extract obtained from *Humulus lupulus* and/or *Angelica keiskei* koidz.

8. The food, beverage or feed according to claim 7, **characterized in that** the pulverized product and/or extract obtained from *Humulus lupulus* comprises xanthohumol and/or a pharmacologically acceptable salt thereof in a high content.

9. A food, beverage or feed for enhancing nerve growth factor production, **characterized in that** the food, beverage or feed comprises as an effective ingredient xanthohumol and/or a pharmacologically acceptable salt thereof.

10. An agent for improving or ameliorating learning and memorizing ability, **characterized in that** the agent comprises a pulverized product and/or extract obtained from *Humulus lupulus* and/or *Angelica keiskei* koidz.

11. The agent according to claim 10, **characterized in that** the pulverized product and/or extract obtained from *Humulus lupulus* comprises xanthohumol and/or a pharmacologically acceptable salt thereof in a high content.

12. An agent for improving or ameliorating learning and memorizing ability, **characterized in that** the agent comprises as an effective ingredient xanthohumol and/or a pharmacologically acceptable salt thereof.

13. A food, beverage or feed for improving or ameliorating learning and memorizing ability, **characterized in that** the food, beverage or feed comprises a pulverized product and/or extract obtained from *Humulus lupulus* and/or *Angelica keiskei* koidz.

14. The food, beverage or feed according to claim 13, **characterized in that** the pulverized product and/or extract obtained from *Humulus lupulus* comprises xanthohumol and/or a pharmacologically acceptable salt thereof in a high content.

15. A food, beverage or feed for improving or ameliorating learning and memorizing ability, **characterized in that** the food, beverage or feed comprises xanthohumol and/or a pharmacologically acceptable salt thereof.
